# EUROPÄISCHE PATENTSCHRIFT

(11) **EP 0 456 602 B1**
(45) Veröffentlichungstag und Bekanntmachung des Hinweises auf die Patenterteilung: **22.02.1995**
(21) Anmeldenummer: 91810303.7
(22) Anmeldetag: 23.04.1991
(51) Int. Cl.: C07D 301/27, C07D 303/36, C08G 59/50

(54) **Verfahren zur Herstellung von N,N,N',N'-Tetraglycidyl-3,3'-dialkyl-4,4'-diaminodiphenylmethanen**
Process for preparation of N,N,N',N'-tetraglycidyl-3,3'-dialkyl-4,4'-diaminophenylmethanes
Procédé pour la préparation des N,N,N',N'-tétraglycidyl-3,3'-dialkyl-4,4'-diaminophénlyméthanes

(30) Priorität: 07.05.1990 CH 1537/90
(43) Veröffentlichungstag der Anmeldung: 13.11.1991
(73) Patentinhaber: CIBA-GEIGY AG, 4002 Basel (CH)
(72) Erfinder: Schaffner, Werner, CH-4125 Riehen (CH)

(56) Entgegenhaltungen:
- EP-A- 0 031 435
- EP-A- 0 287 249
- PATENT ABSTRACTS OF JAPAN vol. 8, no. 34 (C-210)(1471) 15 Februar 1984,& JP-A-
- 58 198475 (KANEGAFUCHI KAGAKU KOGYO K.K.) 18 November 1983,
- PATENT ABSTRACTS OF JAPAN vol. 7, no. 285 (C-201)(1430) 20 Dezember 1983,& JP-A-58 162584 (MITSUBISHI GAS KAGAKU K.K.) 27 September 1983,
- PATENT ABSTRACTS OF JAPAN vol. 8, no. 178 (C-238)(1615) 16 August 1984,& JP-A-59 73577 (MITSUBISHI YUKA K.K) 25 April 1984,

## Beschreibung

Die vorliegende Erfindung betrifft ein Verfahren zum Herstellen von N,N,N',N'-Tetraglycidyl-3,3'-dialkyl-4,4'-diaminodiphenylmethanen, sowie die Verwendung der so erhaltenen Verbindungen zum Herstellen gehärteter Produkte.

Es sind verschiedene Verfahren zur Herstellung von N-Glycidylaminen bekannt.

So wird z.B. in der GB-A-2 111 977 ein Verfahren beschrieben, welches mit Trifluormethansulfonsäure als Katalysator N-Glycidylamine in relativ schlechter Ausbeute und mit tiefem Epoxidgehalt sowie mit hoher Viskosität liefert.

Versuche zeigen, dass die Umsetzung von aromatischen Aminen mit Epichlorhydrin grundsätzlich auch ohne Katalysatoren möglich ist. Die derart hergestellten N-Glycidylamine besitzen aber Nachteile, die ihren Einsatz einschränken und häufig verunmöglichen. Erstens liegt der Epoxidgehalt der so erhaltenen Produkte selten in der Nähe der theoretischen Werte für vollständige Glycidylierung, d.h. der Werte, die man bestimmen würde, falls jedes Aminwasserstoffatom durch eine Glycidylgruppe ersetzt wäre. Der tatsächliche Epoxidgehalt variiert je nach Art des Amins, und ist insbesondere davon abhängig, ob andere Substituenten im Molekül vorhanden sind. So wird z.B. der Epoxidgehalt des handelsüblichen glycidylierten Bis-(aminophenyl)methans in der Kirk-Othmer "Encyclopedia of Chemical Technology", 3. Auflage, Band 9, Seite 277 mit 117-133 angegeben. Dies entspricht einem Epoxidgehalt von 79-90 % des theoretisch möglichen Wertes. Es ist bekannt, dass die Eigenschaften des gehärteten Harzes vom Epoxidgehalt des nicht vernetzten Harzes abhängen: je höher der Epoxidgehalt, desto grösser die Vernetzungsdichte und folglich desto stärker das vernetzte Harz. Es ist eindeutig, dass ein höherer Epoxidgehalt des Harzes von Vorteil wäre.

Der zweite Nachteil von konventionell hergestellten N-Glycidylaminen ist, dass sie oft sehr viskos sind, wahrscheinlich als Folge einer Nebenreaktion während der Herstellung, in der eine Kupplungsreaktion statt der gewünschten Glycidylierung sattfindet. Solche Kupplungsreaktionen verursachen auch die erwähnten tieferen Epoxidgehalt-Werte. Die Verwendung viskoser Harze verursacht Schwierigkeiten, insbesondere bei der Herstellung von faserverstärkten Verbundstoffen oder Giesskörpern, wodurch oft die Anwendung von inerten Verdünnem, die die Viskosität reduzieren, nötig wird.

Im allgemeinen wird die Verwendung von Verdünnern als unerwünscht erachtet. Reaktive Verdünner sind solche, die mit dem Härter reagieren und im vernetzten Harz verbleiben. Sie können sich nachteilig auf die Eigenschaften des gehärteten Harzes auswirken. Inerte Verdünner werden vor dem Härten durch Verdampfung entfernt, und stellen oft eine Gefahr wegen ihrer Brennbarkeit oder Giftigkeit dar. Ausserdem können sie die Eigenschaften des gehärteten Harzes beeinträchtigen, falls sie nicht vollständig aus dem Harz entfernt werden.

Es wurde daher nach Verfahren gesucht, welche N-Glycidylamine ergeben, die die genannten Nachteile nicht oder in weit geringerem Masse aufweisen. In der EP-B 0 143 075 wird ein Verfahren beschrieben, durch welches Produkte mit höherem Epoxidgehalt und niedrigerer Viskosität erhalten werden. Dabei werden als Katalysatoren zwei- oder mehrwertige Metallsalze der Salpetersäure oder der Perchlorsäure, oder zwei- oder mehrwertige Metallsalze einer halogen-enthaltenden Carbonsäure oder Sulfonsäure eingesetzt.

In Patents Abstracts of Japan, Vol. 8, No. 178 (C-238)[1615] August 16, 1984 wird die Herstellung von aromatischen Glycidylverbindungen beschrieben, wobei die Umsetzung von halogen- oder alkyldisubstituierten Diaminodiphenylmethanen mit Epichlorhydrin bei 70-90°C durchgeführt wird und anschliessend die Dehydrohalogenierung des Additionsproduktes mittels Alkalihydroxid in Gegenwart eines quaternären Ammoniumsalzes oder eines Phosphoniumsalzes vorgenommen wird.

Es wäre wünschenswert, ein Verfahren bereitzustellen, bei welchem der Einsatz von Katalysatoren vermieden werden kann, einerseits aus oekologischen und oekonomischen Gründen, und andererseits, weil im genannten Verfahren bei der Aufarbeitung eine unerwünschte Schlammbildung auftritt. Die geringe Selektivität der oben beschriebenen katalysatorenfreien Verfahren ist nicht geeignet, Anhaltspunkte für eine befriedigende Problemlösung zu finden.

Erfindungsgemäss wurde gefunden, dass es möglich ist, bei geeigneter Prozessführung auf die Anwesenheit eines Katalysators zu verzichten.

Die vorliegende Erfindung betrifft ein Verfahren zum Herstellen von Verbindungen der Formel I 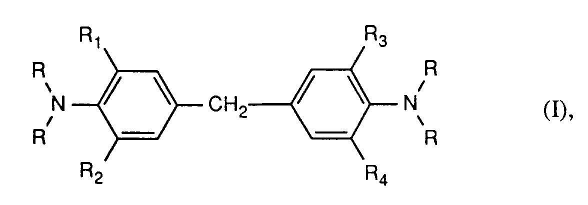
worin
R Glycidyl bedeutet, und
R₁ und R₃ unabhängig voneinander C₁-C₆-Alkyl sind, und
R₂ und R₄ unabhängig voneinander Wasserstoff oder Methyl sind,
dadurch gekennzeichnet, dass man
(i) ein Diamin der Formel II 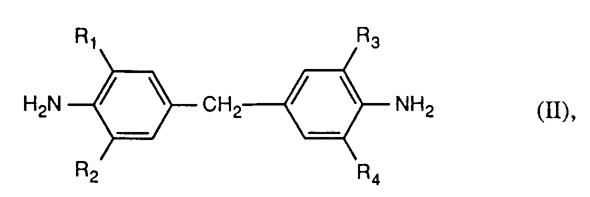 worin R₁, R₂, R₃ und R₄ die oben angegebene Bedeutung haben, mit Epichlorhydrin in einem Molverhältnis von 1:12 bis 1:40 bei einer Temperatur von 80 bis 115°C umsetzt und
(ii) dem Reaktionsgemisch einen Phasentransfer-Katalysator beimischt, das Epichlorhydrin einer Kreislaufdestillation unterwirft und gleichzeitig die Dehydrochlorierung unter Zugabe einer konzentrierten, wässrigen (Erd)Alkalihydroxidlösung und gleichzeitiger Entfernung des azeotrop abdestillierten Wassers durchführt.

Die Diamine der Formel II sind bekannte Verbindungen und z.B. in den US-A-3,427,282 oder 3,560,443 beschrieben.

In bevorzugten Verbindungen der Formel I haben die Substituenten R₁ und R₃ bzw. R₂ und R₄ die gleiche Bedeutung. Besonders bevorzugt sind Verbindungen der Formel I worin R₂ und R₄ Wasserstoff sind und solche, worin R₁ und R₃ Ethyl bedeutet.

Die Additionsreaktion der Stufe (i) erfolgt optimal, d.h. es sind kaum Nebenreaktionen feststellbar. Der Ueberschuss an Epichlorhydrin ist unkritisch, aus oekonomischen Ueberlegungen wurde er aber auf 1:40, bevorzugt 1:20 festgelegt. Für die Reaktionsführung bedeutet dies, dass Epichlorhydrin als Lösungsmittel eingesetzt wird. Dies hat den Vorteil, dass auf ein zusätzliches Lösungsmittel verzichtet werden kann, welche bei den oben erwähnten Katalysatoren-Verfahren zum Einsatz kommen.

Die Reaktionstemperatur der Stufe (i) ist begrenzt durch den Siedepunkt von Epichlorhydrin (118°C) und beträgt 80-115°C, bevorzugt 90-105°C. In den genannten Bereichen ist die Selektivität besonders günstig. Bei Temperaturen unterhalb 80°C müssen ausserdem ungebührend lange Reaktionszeiten in Kauf genommen werden.

Die Reaktionszeiten der Stufe (i) betragen je nach gewählter Reaktionstemperatur im allgemeinen 5 bis 15 Stunden.

Zur Dehydrohalogenierung werden in der Regel starke Alkalien verwendet. Vorzugsweise setzt man wässrige Natronlauge ein, doch können auch andere alkalische Reagentien, wie Kaliumhydroxid, Bariumhydroxid, Calciumhydroxid, Natriumcarbonat oder Kaliumcarbonat, Verwendung finden. Vorzugsweise verwendet man beim erfindungsgemässen Verfahren 20 bis 100 gewichts-%ige Natronlauge. Im allgemeinen arbeitet man bei der Dehydrohalogenierung mit stöchiometrischen Mengen, d.h. 4 Mol (Erd)Alkalihydroxid bezogen auf das Diamin der Formel II, doch ist es zweckmässig, die Halogenwasserstoffabspaltung unter Verwendung eines bis zu 25 %-igen Ueberschusses (5 Mol) über die stöchiometrisch erforderliche Menge an Alkali durchzuführen.

In der Stufe (ii) wird für die Dehydrochlorierung ein Phasentransferkatalysator eingesetzt. Geeignet sind Phasenumwandlungskatalysatoren, wie quaternäre Ammoniumsalze, beispielsweise Tetramethylammoniumchlorid, Tetraäthylammoniumchlorid, Benzyltrimethylammoniumchlorid, Benzyltrimethylammoniumacetat, Methyltriäthylammoniumchlorid, Tetrabutylammoniumchlorid oder Tetrabutylammoniumsulfat, oder die entsprechenden Phosphoniumsalze; quaternäre Ammoniumbasen, beispielsweise Benzyltrimethylammoniumhydroxid, sowie Kronenäther, beispielsweise 12-Kronen-4-äther (1,4,7,10-Tetraoxacyclododecan), 15-Kronen-5-äther (1,4,7,10,13-Pentaoxacyclopentadecan), 18-Kronen-6-äther oder Bibenzo-18-kronen-6-äther. Ferner eignen sich als Katalysatoren tertiäre Amine, wie beispielsweise 2,4,6-Tris-(dimethylaminomethyl)-phenol, Benzyldimethylamin, 1-Methylimidazol, 2-Aethyl-4-methylimidazol oder Aminopyridin.

Bevorzugt werden tertiäre Ammonium- oder Phosphoniumsalze. Diese werden in den üblichen Mengen, z.B. 0,5-10, vorzugsweise 1,5 Mol-%, bezogen auf das Diamin der Formel II eingesetzt.

Es wurde nun gefunden, dass in einer bevorzugten Ausführungsform des erfindungsgemässen Verfahrens in der Stufe (ii) eine schwache anorganische Base mitverwendet wird. Es handelt sich dabei beispielsweise um Hydrogencarbonate des Natriums oder Kaliums. Bevorzugt wird Alkalihydrogencarbonat, insbesondere Natriumhydrogencarbonat eingesetzt. Geeignete Mengen sind 0,1-10 Mol-%, bevorzugt 0,5-5 Mol-%, bezogen auf das Diamin der Formel II.

Die Reaktionsstufe (ii) erfolgt vorzugsweise bei reduziertem Druck, zweckmässigerweise unter azeotropen Bedingungen bei Temperaturen zwischen 30 und 70°C, bevorzugt 40 und 55°C. Dabei ist der Druck so zu wählen, dass Epichlorhydrin und Wasser azeotrop destilliert werden können. Während das Wasser laufend über einem Wasserabscheider aus dem System entfernt wird, wird das Epichlorhydrin der Reaktionsmischung wieder zugeführt (Kreislaufdestillation).

Gleichzeitig zu dieser Kreislaufdestillation und dem laufenden Entfernen des Wassers wird die (Erd)Alkalihydroxid-Lösung langsam zugegeben. Die Zugabe erfolgt zweckmässigerweise gleichmässig in einer Zeitspanne von ≧ 3 Stunden unter kräftigem Rühren.

Die Aufarbeitung erfolgt an sich auf bekannte Weise. Als besonders vorteilhaft hat sich die Wasserextraktion herausgestellt. Dabei gelingt es, zu besonders sauberen Produkten mit einer fast quantitativen Ausbeute zu kommen.

Die nach dem vorliegenden Verfahren erhaltenen N-Glycidylgruppen-enthaltenden Epxoxidharze können auf übliche Weise gehärtet werden. Geeignete Härtungsmittel für N-Glycidylgruppenenthaltende Epoxidharze sind wohl bekannt: sie umfassen z.B. Dicyandiamid, aromatische Amine, wie Bis(3-aminophenyl)- und Bis(4-aminophenyl)sulfon und Bis(4-aminophenyl)methan (meistens zusammen mit einem Härtungsbeschleuniger, wie z.B. einem BF₃-Amin-Komplex), sowie Anhydride von Polycarbonsäuren, wie Cyclohexan-1,2-dicarbonsäureanhydrid, Methylbicyclo[2.2.1]-hept-5-en-2,3-dicarbonsäureanhydrid, und Benzophenontetracarbonsäuredianhydrid.

Beispiel 1: In einem 1500 ml Reaktionskolben mit Untenauslauf werden 1300 g Epichlorhydrin unter starkem Rühren auf 98-100°C aufgeheizt und innerhalb von 10 min. 85 g 3,3'-Diethyl-4,4'-diaminodiphenylmethan zudosiert. Nach weiteren 10 min. werden bei der gleichen Temperatur während 60 min. 169,3 g 3,3'-Diethyl-4,4'-diaminodiphenylmethan zugegeben. Die Reaktionslösung wird während 6½ h bei 98-100°C gerührt und anschliessend auf 50°C abgekühlt. Darauf werden 5 g Tetramethylammoniumchlorid als 50%-ige wässrige Lösung und 15 g Natriumhydrogencarbonat zugegeben. Mittels eines Vakuums von ca. 85 mbar wird Epichlorhydrin im Kreislauf über einen Wasserabscheider bei ca. 44-50°C Innentemperatur destilliert und gleichzeitig während 300 min. 328 g einer 50%-igen wässrigen Lösung von Natriumhydroxid gleichmässig zudosiert. Nach der Zugabe wird 30 min. weiter destilliert und darauf auf 35°C gekühlt. Bei 35°C werden 700 g Wasser zugegeben und nach 5 min. Rühren zur Phasentrennung stehen gelassen. Die untere wässrige Salzlösung wird nach 15 min. abgetrennt. Zur organischen Phase im Reaktor werden 200 g Wasser und eine 50%-ige wässrige Lösung von 5 g Natriumhydrogensulfat zugegeben, 5 min. gerührt und 15 min. zur Phasentrennung stehen gelassen. Die untere organische Phase wird abgetrennt und in einem Trenngefäss mit 200 g Wasser extrahiert. Nach dem Abtrennen wird das überschüssige Epichlorhydrin bei einer Temperatur bis 120°C und unter Vakuum abdestilliert. Zum Schluss werden die flüchtigen Anteile mit 30 g Wasser gestrippt und das Produkt 60 min. bei 120°C getrocknet, auf 80°C gekühlt und mit 5 g Filterhilfsmittel Celatom 80 über ein Suprafilter 200 filtriert.

Die Ausbeute beträgt etwa 98 % der Theorie, bezogen auf 3,3'-Diethyl-4,4'-diaminodiphenylmethan. Der Epoxidgehalt entspricht 7,95 Val/kg, das verseifbare Chlor etwa 400 ppm, und die Viskosität nach Höppler bei 25°C 9000 mPa·s.

Beispiel 2: Es wird analog vorgegangen wie in Beispiel 1 beschrieben, wobei die Zugabe des 3,3'-Diethyl-4,4'-diaminodiphenylmethans in einer Portion erfolgt. Dabei werden praktisch die gleichen Ergebnisse wie in Beispiel 1 erzielt.

Beispiel 3: Beispiel 1 wird wiederholt, indem das dort verwendete Tetramethylammoniumchlorid durch Tetrabutylammoniumchlorid ersetzt wird. Das erhaltene Epoxidharz hat einen Epoxidgehalt von 7,75 Val/kg, einen Gehalt an verseibaren Chlor von etwa 100 ppm und eine Viskosität nach Höppler bei 25°C von 9200 mPa·s.

Beispiel 4: Beispiel 2 wird wiederholt, indem man das dort verwendete 3,3'-Diethyl-4,4'-diaminodiphenylmethan durch 282 g 4,4'-Methylen-bis-(2-methyl-6-ethylanilin) ersetzt. So erhält man ein Epoxidharz mit einem Epoxidgehalt von 7,3 Val/kg und einer Viskosität nach Höppler bei 25°C von 48700 mPa·s.

Beispiel 5: Beispiel 2 wird wiederholt, indem man das dort verwendete 3,3'-Diethyl-4,4'-diaminodiphenylmethan durch 310 g 4,4'-Methylen-bis-(2,6-diethylanilin) ersetzt. Man erhält ein Epoxidharz mit einem Epoxidgehalt von 6,09 Val/kg und einer Viskosität nach Höppler bei 25°C von 40500 mPa·s. Das Epoxidharz neigt zur Kristallisation.

## Patentansprüche

1. Verfahren zum Herstellen von Verbindungen der Formel I 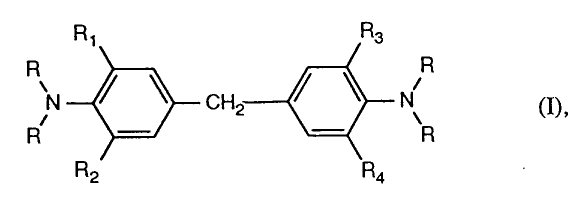 worin
R Glycidyl bedeutet, und
R₁ und R₃ unabhängig voneinander C₁-C₆-Alkyl sind, und
R₂ und R₄ unabhängig voneinander Wasserstoff oder Methyl sind,
dadurch gekennzeichnet, dass man
(i) ein Diamin der Formel II 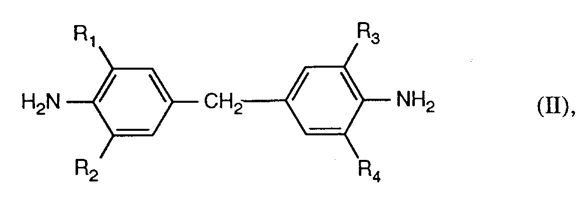 worin R₁, R₂, R₃ und R₄ die oben angegebene Bedeutung haben mit Epichlorhydrin in einem Molverhältnis von 1:12 bis 1:40 bei einer Temperatur von 80 bis 115°C umsetzt und
(ii) dem Reaktionsgemisch einen Phasentransfer-Katalysator und eine schwache anorganische Base bei 30-70°C beimischt, das Epichlorhydrin einer Kreislaufdestillation unterwirft und gleichzeitig die Dehydrochlorierung unter Zugabe einer konzentrierten, wässrigen (Erd)Alkalihydroxidlösung und gleichzeitiger Entfernung des azeotrop abdestillierten Wassers durchführt.

2. Verfahren gemäss Anspruch 1, dadurch gekennzeichnet, dass in der Stufe (i) das Molverhältnis Diamin der Formel II zu Epichlorhydrin 1:12 bis 1:20 beträgt.

3. Verfahren gemäss Anspruch 1, dadurch gekennzeichnet, dass in der Stufe (i) die Temperatur 90-105°C beträgt.

4. Verfahren gemäss Anspruch 1, dadurch gekennzeichnet, dass pro Mol Diamin der Formel II in der Stufe (ii) 4-5 Mol (Erd)Alkalihydroxid verwendet werden.

5. Verfahren gemäss Anspruch 1, dadurch gekennzeichnet, dass in der Stufe (ii) der Phasentransfer-Katalysator ein tertiäres Ammonium- oder Phosphoniumsalz ist.

6. Verfahren gemäss Anspruch 1, dadurch gekennzeichnet, dass die Stufe (ii) bei reduziertem Druck durchgeführt wird.

7. Verfahren gemäss Anspruch 1, dadurch gekennzeichnet, dass in der Stufe (ii) die Temperatur 40-55°C beträgt.

8. Verfahren gemäss Anspruch 1, dadurch gekennzeichnet, dass als schwache Base ein Alkalihydrogencarbonat verwendet wird.

9. Verfahren gemäss Anspruch 8, dadurch gekennzeichnet, dass als schwache Base ein Na- oder K-hydrogencarbonat verwendet wird.

10. Verfahren gemäss Anspruch 8, dadurch gekennzeichnet, dass als schwache Base ein Na-hydrogencarbonat verwendet wird.

11. Verfahren gemäss Anspruch 1, dadurch gekennzeichnet, dass in Formel (I) die Substituenten R₁ und R₃ bzw. R₂ und R₄ die gleiche Bedeutung haben.

12. Verfahren gemäss Anspruch 1, dadurch gekenzeichnet, dass in Formel (I) die Substituenten R₂ und R₄ Wasserstoff bedeuten.

13. Verfahren gemäss Anspruch 1, dadurch gekennzeichnet, dass in der Formel I die Substituenten R₁ und R₃ Ethyl bedeuten.

## Claims

1. A process for the preparation of a compound of the formula I 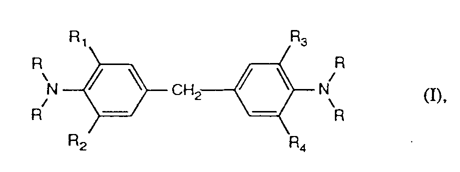 in which R is glycidyl, R₁ and R₃ independently of one another are C₁-C₆alkyl and R₂ and R₄ independently of one another are hydrogen or methyl,
wherein
(i) a diamine of the formula II 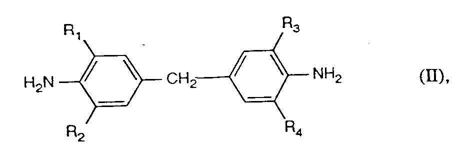 in which R₁, R₂, R₃ and R₄ are as defined above, is reacted with epichlorohydrin in a molar ratio of 1:12 to 1:40 at a temperature of 80 to 115°C and
(ii) a phase transfer catalyst and a weak organic base are mixed with the reaction mixture at 30-70°C, the epichlorohydrin is subjected to distillation while being circulated and at the same time the dehydrochlorination is carried out with the addition of a concentrated, aqueous alkali metal or alkaline earth metal hydroxide solution and simultaneous removal of the water distilled off azeotropically.

2. A process according to claim 1, wherein, in stage (i), the molar ratio of diamine of the formula II to epichlorohydrin is 1:12 to 1:20.

3. A process according to claim 1, wherein, in stage (i), the temperature is 90-105°C.

4. A process according to claim 1, wherein 4-5 mol of alkali metal or alkaline earth metal hydroxide are used per mol of diamine of the formula II in stage (ii).

5. A process according to claim 1, wherein, in stage (ii), the phase transfer catalyst is a tertiary ammonium or phosphonium salt.

6. A process according to claim 1, wherein stage (ii) is carried out at reduced pressure.

7. A process according to claim 1, wherein, in stage (ii), the temperature is 40-55°C.

8. A process according to claim 1, wherein the weak base used is an alkali metal bicarbonate.

9. A process according to claim 8, wherein the weak base used is a sodium or potassium bicarbonate.

10. A process according to claim 8, wherein the weak base used is a sodium bicarbonate.

11. A process according to claim 1, wherein, in formula (I), the substituents R₁ and R₃ or R₂ and R₄ have the same meaning.

12. A process according to claim 1, wherein, in formula (I), the substituents R₂ and R₄ are hydrogen.

13. A process according to claim 1, wherein, in formula (I), the substituents R₁ and R₃ are ethyl.

## Revendications

1. Procédé pour la préparation de composés de formule I 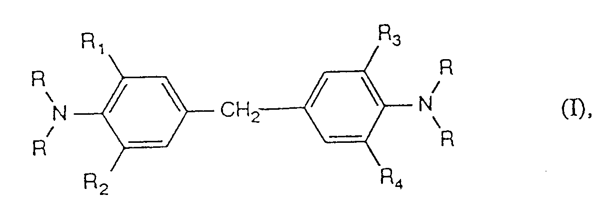 dans laquelle
R représente le glycidyle, et
R₁ et R₃, indépendamment l'un de l'autre, sont un alkyle en C₁-C₆, et
R₂ et R₄, indépendamment l'un de l'autre, sont l'hydrogène ou le méthyle,
caractérisé en ce qu'on fait réagir :
(i) une diamine de formule II 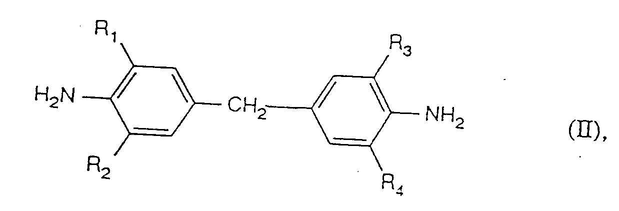 dans laquelle R₁, R₂, R₃ et R₄ ont les significations données ci-dessus, avec l'épichlorhydrine dans un rapport molaire de 1:12 à 1:40, à une température de 80 à 115°C, et
(ii) on ajoute par mélange au mélange réactionnel un catalyseur de transfert de phases et une base minérale faible à une température de 30 à 70°C, on soumet l'épichlorhydrine à une distillation par recirculation et on effectue simultanément la déshydrochloration en ajoutant une solution concentrée aqueuse d'hydroxyde alcalin (alcalino-terreux) et en éliminant simultanément l'eau par distillation azéotropique.

2. Procédé selon la revendication 1, caractérisé en ce que dans l'étape (i), le rapport molaire de la diamine de formule II à l'épichlorhydrine est de 1:12 à 1:20.

3. Procédé selon la revendication 1, caractérisé en ce que dans l'étape (i) la température est de 90 à 105°C.

4. Procédé selon la revendication 1, caractérisé en ce qu'on utilise par mole de diamine de formule II dans l'étape (ii) de 4 à 5 moles d'hydroxyde alcalin (alcalino-terreux).

5. Procédé selon la revendication 1, caractérisé en ce que le catalyseur de transfert de phases dans l'étape (ii) est un sel de phosphonium ou d'ammonium tertiaire.

6. Procédé selon la revendication 1, caractérisé en ce qu'on effectue l'étape (ii) sous pression réduite.

7. Procédé selon la revendication 1, caractérisé en ce que dans l'étape (ii) la température est de 40 à 55°C.

8. Procédé selon la revendication 1, caractérisé en ce qu'on utilise en tant que base faible un bicarbonate alcalin.

9. Procédé selon la revendication 8, caractérisé en ce qu'on utilise en tant que base faible un bicarbonate de Na ou de K.

10. Procédé selon la revendication 8, caractérisé en ce qu'on utilise en tant que base faible un bicarbonate de Na.

11. Procédé selon la revendication 1, caractérisé en ce que dans la formule (I) les substituants R₁ et R₃, respectivement R₂ et R₄, ont la même signification.

12. Procédé selon la revendication 1, caractérisé en ce que dans la formule (I) les substituants R₂ et R₄ représentent l'hydrogène.

13. Procédé selon la revendication 1, caractérisé en ce que dans la formule I les substituants R₁ et R₃ représentent l'éthyle.
